# EUROPEAN PATENT APPLICATION

(11) **EP 2 873 445 A1**
(43) Date of publication of application: **20.05.2015**
(21) Application number: 14192997.6
(22) Date of filing: 13.11.2014
(51) Int. Cl.: A63B 69/36, G09B 19/00

(54) **Motion analyzing method and motion analyzing apparatus**

(30) Priority: 15.11.2013 JP 2013236598
(71) Applicant: Seiko Epson Corporation, Tokyo 163 (JP)
(72) Inventor: Shibuya, Kazuhiro, Nagano, 392-8502 (JP)
(74) Representative: Hoffmann Eitle

(57) **Abstract**

A motion analyzing method includes: acquiring location information of at least one of a sporting tool and a subject in swing motion based on an inertial force generated by the swing motion in time series; acquiring analysis information of the swing motion based on the inertial force; acquiring a time when the analysis information is switched with respect to a preset threshold value; and correlating information of the time with the location information and outputting the information.

## Description

### BACKGROUND

### 1. Technical Field

The present invention relates to a motion analyzing method and a motion analyzing apparatus.

### 2. Related Art

A motion analyzing apparatus is used for analysis of motion including swing motion. The position of a sporting tool at swing changes over time. The swing motion is visually reproduced by trace display or the like based on the output of an inertia sensor attached to the sporting tool or a hand. As one specific example of the motion analyzing apparatus, for example, there is an example disclosed in Patent Document 1 (JP-A-2011-142927). In this example, swing trace information and an analysis result are separately displayed as disclosed in Fig. 12 of Patent Document 1, and the analysis result is displayed with two axes of "time on horizontal axis" and "analysis information on vertical axis" as shown in Fig. 11 of Patent Document 1. The method of displaying the analysis information with two axes is often used.

However, according to the method of displaying the analysis information with two axes as described above, it is difficult to cross-check the point of the swing motion and the analysis result in time series at a glance. For example, there are problems that it is difficult for a golfer to grasp the times when the state changes of the swing (e.g., changes of the face and changes of swing energy) occur, the bad points of the swing motion, or the like.

### SUMMARY

An advantage of some aspects of the invention is to solve at least a part of the problems described above, and the invention can be implemented as the following forms or application examples.

### Application Example 1

This application example is directed to a motion analyzing method including: acquiring location information of at least one of a sporting tool and a subject in swing motion in time series based on an inertial force generated by the swing motion, acquiring analysis information of the swing motion based on the inertial force, acquiring a time when the analysis information is switched with respect to a preset threshold value, and correlating information of the time with the location information and outputting the information.

According to this application example, the information of the time is correlated with the location information of the swing trace or the like and output, and thereby, for example, a golfer may grasp at a glance the time when the state change occurs in the swing, at which point of the motion the swing is bad (good), or the like and a motion analysis result with advantageous convenience may be provided to the golfer.

### Application Example 2

In the motion analyzing method according to the application example described above, it is preferable that the outputting includes correlating the information of the time with a trace of the swing motion drawn in time series based on the location information and outputting the information.

### Application Example 3

In the motion analyzing method according to the application example described above, it is preferable that the displaying includes changing display of the trace between before and after the time.

According to this application example, the display of the location information (swing trace or the like) is changed between before and after the time when the analysis information is switched with respect to the threshold value, and thereby, the time when the quality or the state of the analysis data is changed may be grasped at a glance during the swing motion.

### Application Example 4

In the motion analyzing method according to the application example described above, it is preferable that analysis information is position data of the sporting tool in the swing motion.

According to this application example, for example, the position data of the sporting tool may be displayed with the swing trace as the location information in the swing motion, and thus, as the state of the position of the sporting tool, e.g., the position data of the sporting tool, the amount of change of the rotation angle around the extending longitudinal axis of the shaft (shaft rotation angle) from address to impact, the analysis data of the tilt angle of the shaft, or the like may be grasped in time series with the swing trace. As described above, the analysis data representing the state of the position of the sporting tool may be grasped at a glance with the swing trace, and the state of the swing motion and the quality tendency may be precisely determined.

### Application Example 5

In the motion analyzing method according to the application example described above, it is preferable that the motion analyzing method includes determining a state change of swing before and after the time, and the outputting includes changing display of the trace in response to the state change.

According to this application example, whether or not the state of the swing is changed between before and after the analysis information calculated in time series exceeds the threshold value is determined, the state change is represented by e.g., gradation display or the like in the swing trace, and thereby, the golfer may precisely determine the time when the state change occurs during swing.

### Application Example 6

In the motion analyzing method according to the application example described above, it is preferable that the motion analyzing method includes determining quality of swing before and after the time, and the outputting includes changing display of the trace in response to the quality.

According to this application example, the quality of the swing is changed between before and after the analysis information calculated in time series exceeds the threshold value is determined, the quality is represented by e.g., gradation display or the like in the swing trace, and thereby, the golfer may precisely determine the time when the swing is bad (good) during swing.

### Application Example 7

This application example is directed to a motion analyzing method including: acquiring location information of at least one of a sporting tool and a subject in swing motion in time series based on an inertial force generated by the swing motion, acquiring analysis information of the swing motion based on the inertial force and generating a graph of the analysis information, and correlating information of the graph with a trace of the swing motion drawn in time series based on the location information and outputting the information.

According to this application example, for example, the graph obtained from the analysis information is displayed with the swing trace display. The graph can be displayed in thin lines, and thereby, a plurality of pieces of analysis information may be superimposed and displayed and visible recognition may be improved.

### Application Example 8

This application example is directed to a motion analyzing apparatus including: a unit that acquires location information of at least one of a sporting tool and a subject in swing motion in time series based on an inertial force generated by the swing motion, a unit that acquires analysis information of the swing motion based on the inertial force, a unit that acquires a time when the analysis information is switched with respect to a preset threshold value, and a unit that correlates information of the time with the location information and outputs the information.

According to this application example, for example, the analysis information is displayed with the location information of the swing trace or the like in time series, and thereby, a golfer may grasp at a glance the time when the state change occurs in the swing, at which point of the motion the swing is bad (good), or the like and a motion analysis result with advantageous convenience may be provided to the golfer.

### Application Example 9

In the motion analyzing apparatus according to the application example described above, it is preferable that the motion analyzing apparatus includes a unit that correlates the information of the time with a trace of the swing motion drawn in time series based on the location information and outputs the information.

### Application Example 10

In the motion analyzing apparatus according to the application example described above, it is preferable that display of the trace is changed between before and after the time.

According to this application example, the display of the location information (swing trace or the like) is changed between before and after the time when the analysis information is switched with respect to the threshold value, and thereby, the time when the quality or the state of the analysis data is changed may be grasped at a glance during the swing motion.

### BRIEF DESCRIPTION OF THE DRAWINGS

The invention will be described with reference to the accompanying drawings, wherein like numbers reference like elements.
Fig. 1 is a conceptual diagram schematically showing a configuration of a golf swing analyzing apparatus according to a first embodiment of the invention.
Fig. 2 is a block diagram schematically showing a configuration of an arithmetic processing circuit.
Fig. 3 shows one specific example of a graph showing changes of an energy change rate according to a time axis.
Fig. 4 shows one specific example of a pseudo circular graph that correlates the changes of the energy change rate and a swing trace.
Fig. 5 shows one specific example of an image visually representing a movement trace of a golf club.
Fig. 6 shows one specific example of a graph showing changes of a relative rotation angle according to the time axis of the second embodiment.
Fig. 7 shows one specific example of a pseudo circular graph that correlates the changes and the swing trace according to the second embodiment.
Fig. 8 shows one specific example of a pseudo circular graph that correlates the changes of the energy change rate with the swing trace according to the third embodiment.

### DESCRIPTION OF EXEMPLARY EMBODIMENTS

As below, embodiments of the invention will be explained. The embodiments described below do not unduly limit the invention described in the appended claims, and not all of the configurations explained in the embodiments are not essential as a resolving means of the invention.

### First Embodiment

As below, a golf swing analyzing apparatus (motion analyzing apparatus) and a golf swing analyzing method (motion analyzing method) according to the invention will be explained with reference to the drawings. The golf swing analyzing method (motion analyzing method) will be explained in conjunction with the explanation of the golf swing analyzing apparatus (motion analyzing apparatus).

### (1) Configuration of Golf Swing Analyzing Apparatus

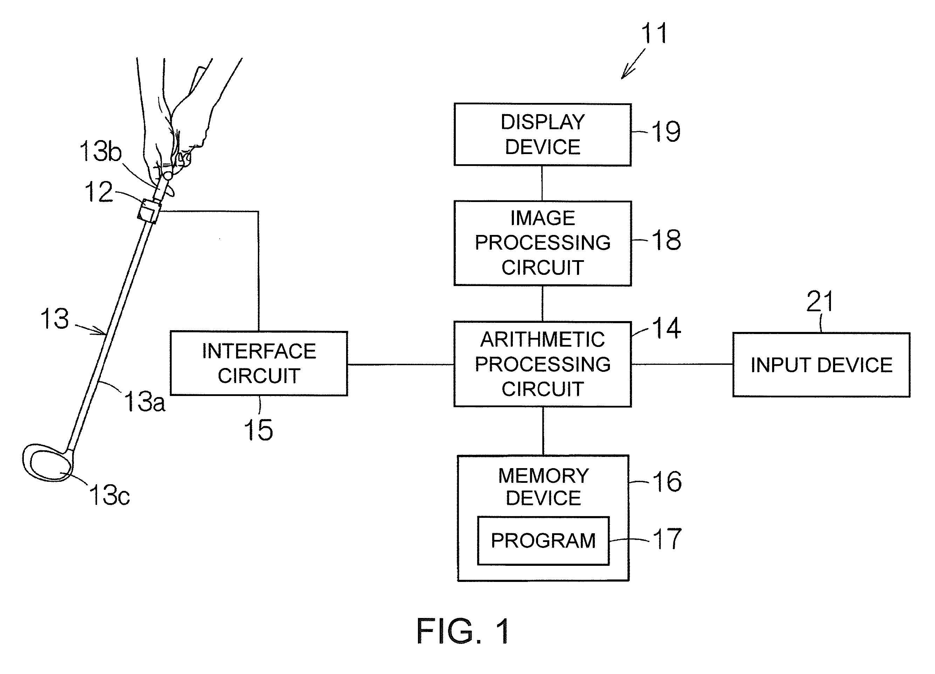

A configuration of the golf swing analyzing apparatus (motion analyzing apparatus) according to the embodiment of the invention is explained with reference to Fig. 1. Fig. 1 schematically shows a configuration of a golf swing analyzing apparatus (motion analyzing apparatus) 11 according to the embodiment of the invention. The golf swing analyzing apparatus 11 includes, e.g. , an inertia sensor 12. For example, an acceleration sensor and a gyro sensor are incorporated in the inertia sensor 12. The acceleration sensor can detect accelerations individually in three axis directions orthogonal to one another. The gyro sensor can detect angular velocities individually around the respective three axes orthogonal to one another. The inertia sensor 12 outputs detection signals. The acceleration and the angular velocity are specified with respect to each axis by the detection signals.

### (2) Golf Swing Analyzing Method

A motion analyzing method according to the first embodiment includes calculating location information of a swing trace in swing motion or the like in time series using output of the inertia sensor 12, calculating an energy change rate in the swing motion in time series as analysis information using the output of the inertia sensor 12, acquiring a time of switching with respect to a preset threshold value (hereinafter, referred to as "switching time"), and displaying the swing trace with the energy change rate. Further, the above described displaying includes changing display of the swing trace with the switching time. Specifically, as will be described later, the changes of the energy change rate in the swing motion obtained as analysis information are drawn on the swing trace with the switching times using gradation display or the like.

The inertia sensor 12 is attached to a golf club (sporting tool) 13. The golf club 13 has a shaft 13a and a grip 13b. The grip 13b is held by hands. The grip 13b is coaxially formed with the longitudinal axis along which the shaft 13a extends. A club head 13c is coupled to the end of the shaft 13a. The inertia sensor 12 is attached to the shaft 13a or the grip 13b of the golf club 13. It is only necessary that the inertia sensor 12 is relatively immovably fixed to the golf club 13. Here, for attachment of the inertia sensor 12, one of the detection axes of the inertia sensor 12 is aligned with the axis of the shaft 13a. Another one of the detection axes of the inertia sensor 12 is desirably aligned with the horizontal direction with respect to the face of the club head 13c.

The golf swing analyzing apparatus 11 includes an arithmetic processing circuit 14. The inertia sensor 12 is connected to the arithmetic processing circuit 14. For the connection, a predetermined interface circuit 15 is connected to the arithmetic processing circuit 14. The interface circuit 15 may be in wired or wireless connection to the inertia sensor 12. The detection signals are supplied from the inertia sensor 12 to the arithmetic processing circuit 14.

A memory deice 16 is connected to the arithmetic processing circuit 14. For example, a golf swing analysis software program (motion analyzing program) 17 and relevant data are stored in the memory device 16. The arithmetic processing circuit 14 executes the golf swing analysis software program 17 to realize a golf swing analyzing method. The memory device 16 includes a DRAM (dynamic random access memory), a mass-storage unit, a nonvolatile memory, etc. For example, the golf swing analysis software program 17 is temporarily held in the DRAM for implementation of the golf swing analyzing method. The golf swing analysis software program 17 and the data are saved in the mass-storage unit such as a hard disc drive (HDD). Programs and data having relatively low capacity such as BIOS (basic input/output system) are stored in the nonvolatile memory.

An image processing circuit 18 is connected to the arithmetic processing circuit 14. The arithmetic processing circuit 14 sends predetermined image data to the image processing circuit 18. A display device 19 is connected to the image processing circuit 18. For the connection, a predetermined interface circuit (not shown) is connected to the image processing circuit 18. The image processing circuit 18 sends image signals to the display device 19 in response to the input image data. Images specified by the image signals are displayed on the screen of the display device 19. A liquid crystal display or another flat panel display is used for the display device 19. Here, the arithmetic processing circuit 14, the memory device 16, and the image processing circuit 18 are provided as a computer device, for example.

An input device 21 is connected to the arithmetic processing circuit 14. The input device 21 includes at least alphabet keys and a numeric key pad. Character information and numeric information including e.g., threshold values are input from the input device 21 to the arithmetic processing circuit 14. The input device 21 may include a keyboard, for example. The combination of the computer device and the keyboard may be replaced by a smartphone, a cell phone terminal, a tablet PC (personal computer), or the like.

### (3) Configuration of Arithmetic Processing Circuit

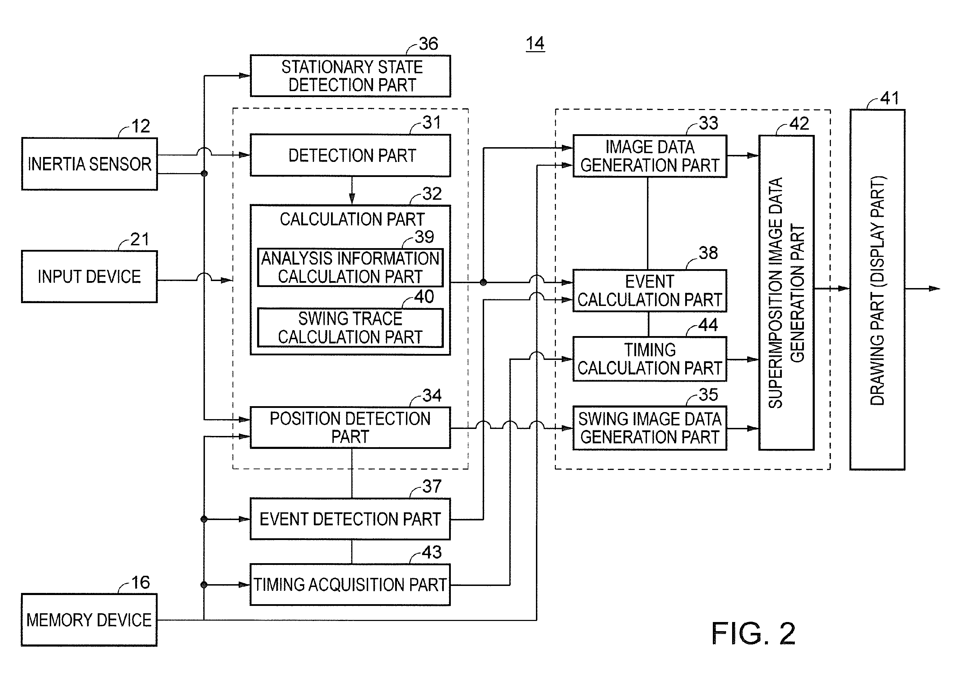

Fig. 2 schematically shows a configuration of the arithmetic processing circuit 14 according to one embodiment. The arithmetic processing circuit 14 includes a detection part 31. The detection part 31 is connected to the inertia sensor 12, and the output is supplied from the inertia sensor 12 to the part.

The detection part 31 detects a first state (initial state) of the grip 13b around an axis of the grip 13b (coaxial with the shaft 13a) based on the output of the inertia sensor 12. For the detection, the detection part 31 acquires the angular velocity around one detection axis (here, around the y-axis) in parallel to the shaft 13a at address by the inertia sensor 12. The detection part 31 sets an initial value to the acquired angular velocity. At address, no angular velocity is generated around the y-axis, and thus, when the club stops moving at the angular velocity of "0 (zero)", the angle "0° (zero degrees) " (= initial state) is set. The position of the shaft 13a at address corresponds to a first state of the shaft part and a stationary state before start of swing motion. Then, the detection part 31 detects a second state of the grip 13b during swing around the axis of the grip 13b in swing motion. Here, the position of the shaft 13a at an event such as halfway-back or top corresponds to the second state of the shaft part. For the detection, the detection part 31 acquires the angular velocity around one detection axis (here, around the y-axis) in parallel to the shaft 13a by the inertia sensor 12 in the swing motion. The detection part 31 sets a value in the second direction to the acquired angular velocity. Regarding the value in the second state, the values may be continuously acquired per unit time from start to end of swing, or a value only at a predetermined time in swing motion (e.g., at impact) may be acquired.

A calculation part 32 is connected to the detection part 31 and the output acquired in the detection part 31 is supplied thereto. The calculation part 32 includes an analysis information calculation part 39 and a swing trace calculation part 40. The analysis information calculation part 39 calculates from an initial state at the angle "0°" to a transfer state of energy generated between the subject and the golf club 13 during swing, in other words, a positive or negative state of application of the energy, or an energy change rate of the state of application of the energy according to the time axis and a relative rotation angle θn of the grip 13b according to the time axis based on the output of the inertia sensor 12. Note that the energy change rate and the relative rotation angle θn of the grip 13b are examples of the analysis information. As below, in the embodiment, the energy change rate shown according to the time axis is exemplified. The energy change rate is calculated by correlation between a first amount of energy generated in the upper limb of the subject and a second amount of energy transferred from the upper limb of the subject to the golf club 13. The energy change rate indicates the first amount of energy that changes depending on the second amount of energy transferred from the upper limb of the subject to the golf club 13. The swing trace calculation part 40 calculates the trace of the swing according to the time axis (swing trace in time series) in the three-dimensional space using the above described motion analysis model based on the output of the inertia sensor 12. Note that the swing trace includes a trace of the shaft and a movement trace of the subject itself.

Furthermore, in the calculation part 32, an evaluation of the quality of the swing state is made using the specified energy change rate. In the evaluation, comparison with a preset threshold value of the energy change rate is made. As the threshold value in this case, the time axis of the axis on which the output signal is zero, in other words, the positive and the negative states of the energy change rate are switched is used. For example, the evaluation is on whether the energy change rate in the swing trace along the time axis is in the positive state or the negative state or shifts toward the positive state or the negative state.

The arithmetic processing circuit 14 includes an image data generation part 33. The image data generation part 33 is connected to the calculation part 32. The output is supplied from the calculation part 32 to the image data generation part 33. The image data generation part 33 generates image data. In the image data, an image visually representing the energy change rate is specified. The image data of the image data generation part 33 specifies an image representing changes of the energy change rate according to the time axis. For example, the image may be a graph in which the time axis is set on the horizontal axis and the energy change rate is set on the vertical axis. Here, the image data may contain data (pattern) superimposed on the image, e.g., swing trace pattern. The data (pattern) superimposed on the image may represent swing motion of a professional, an expert, another subject having the equal skill to the subject, or the like. Further, the energy change rate of the professional, the expert, or the like for comparison with the subject may be superimposed and displayed. Furthermore, the image data generation part 33 may correlate the evaluation result calculated in the calculation part 32 with the image data for visually representing the energy change rate and display them. In this case, for the image data correlated and displayed, graphic display in different colors at times when the positive and negative states of the energy change rate are switched or gradation display with the times when the positive and negative states of the energy change rate are switched may be used.

The image data is used, and thereby, the state of the energy change rate along the swing trace may be visually recognized according to the switching time, and the positive or negative state of the energy change rate or the quality of the analysis data during swing motion may be grasped at a glance. Further, the energy change rate is displayed with the swing trace in the swing motion, and thus, the switching time of the energy provided to the golf club (sporting tool) 13 may be grasped at a glance and the state of the swing motion and the quality tendency may be precisely determined.

The arithmetic processing circuit 14 includes a position detection part 34. The position detection part 34 is connected to the inertia sensor 12. The output is supplied from the inertia sensor 12 to the position detection part 34. Here, the output of the inertia sensor 12 includes the accelerations respectively detected along the orthogonal three axes and the angular velocities respectively detected around the orthogonal three axes. The position detection part 34 detects the position of the golf club 13 based on the output of the inertia sensor 12. For the position detection, the position detection part 34 detects the locations of the grip 13b and the club head 13c in motion. For the location detection, for example, the position detection part 34 calculates the acceleration of the grip 13b. For the acceleration calculation, the position detection part 34 specifies the location lsj of the grip 13b according to an intrinsic local coordinate system Σs of the inertia sensor 12. For the specification, the position detection part 34 acquires location information from the memory device 16. The locations lsj of the grip 13b are stored in the memory device 16 in advance. The location lsj of the grip 13b may be designated via the input device 21, for example.

The position detection part 34 calculates the movement speeds and the locations of the grip 13b based on the calculated accelerations. Similarly, the position detection part 34 detects the locations of the club head 13c. For the location detection, the position detection part 34 specifies the locations lsh of the club head 13c according to the intrinsic local coordinate system Σs of the inertia sensor 12. For the specification, the position detection part 34 acquires location information from the memory device 16. The locations lsh of the club head 13c are stored in the memory device 16 in advance. The location lsh of the club head 13c may be designated via the input device 21, for example.

The arithmetic processing circuit 14 includes a swing image data generation part 35. The swing image data generation part 35 is connected to the position detection part 34. The output of the position detection part 34 is supplied to the swing image data generation part 35. The swing image data generation part 35 specifies the movement trace of the golf club 13 based on the locations of the grip 13b and the locations of the club head 13c calculated in the position detection part 34. An image representing the swing motion is generated based on the specified movement trace. The image is output as image data from the swing image data generation part 35.

The arithmetic processing circuit 14 includes a stationary state detection part 36. The stationary state detection part 36 is connected to the inertia sensor 12. The output is supplied from the inertia sensor 12 to the stationary state detection part 36. Here, the output of the inertia sensor 12 includes the accelerations respectively detected along the orthogonal three axes and the angular velocities respectively detected around the orthogonal three axes. The stationary state detection part 36 determines the stationary state of the golf club 13 based on the output of the inertia sensor 12. When the output of the inertia sensor 12 is below a threshold value, the stationary state detection part 36 determines the stationary state of the golf club 13. The stationary state of the golf club 13 represents address in swing motion. The threshold value may be set to a value that can eliminate the influence of the detection signal indicating minute vibration such as body motion. When confirming the stationary state over a predetermined period, the stationary state detection part 36 outputs a stationary state notification signal. The stationary state notification signal is sent to the detection part 31, the calculation part 32, and the position detection part 34. The detection part 31 sets the initial state at the angle "0°" in response to the reception of the stationary state notification signal, and the calculation part 32 starts calculation of the energy change rate in response to the reception of the stationary state notification signal. The position detection part 34 starts detection of the position of the golf club 13 in response to the reception of the stationary state notification signal.

Here, for determination of the stationary state, the stationary state detection part 36 may refer to the tilt angle of the golf club 13. In this regard, the stationary state detection part 36 calculates the tilt angle, i.e. , the position of the golf club 13 based on the coordinates of the grip 13b and the coordinates of the club head 13c. The stationary state detection part 36 determines the position of the golf club 13 at address based on the calculated tilt angle. Whether or not the tilt angle falls within a predetermined tilt angle range is determined. The position of the golf club 13 at address is established, and then, the stationary state detection part 36 starts determination of the stationary state of the golf club 13.

The arithmetic processing circuit 14 includes an event detection part 37. The event detection part 37 is connected to the position detection part 34. The output of the position detection part 34 is supplied to the event detection part 37. The event detection part 37 specifies the event during swing motion based on the position of the golf club 13. For example, the event detection part 37 detects the axis of the grip 13b (i.e. , the axis of the shaft 13a) provided in parallel to the ground. In this manner, halfway-back during back-swing may be specified. For example, the event detection part 37 may detect the changes of accelerations at switching from back-swing to down-swing. In this manner, top of back-swing is specified. For the detection, the event detection part 37 may acquire a reference value for comparison from the memory device 16.

The arithmetic processing circuit 14 includes an event calculation part 38. The event calculation part 38 is connected to the event detection part 37 and the calculation part 32. The output of the event detection part 37 and the output of the calculation part 32 are supplied to the event calculation part 38. The event calculation part 38 correlates individual events specified in the event detection part 37 with the analysis information. The events such as halfway-back and top are correlated with the specific analysis information.

The arithmetic processing circuit 14 includes a timing acquisition part 43. The timing acquisition part 43 acquires the switching time with respect to the preset threshold value in the analysis information (energy change rates) calculated in time series. That is, of the energy change rates calculated in time series, the part acquires the time when the energy change rate is switched to "positive" or "negative". The threshold value is set to a value when the energy change rate is switched between the positive state and the negative state as described above. That is, the time axis on which the energy change rate is zero is used as the threshold value.

The arithmetic processing circuit 14 includes a timing calculation part 44. The timing calculation part 44 is connected to the timing acquisition part 43 and the event calculation part 38. The output of the timing acquisition part 43 and the output of the event calculation part 38 are supplied to the timing calculation part 44. The timing calculation part 44 correlates the switching times between "positive" and "negative" of the energy change rate (analysis information) with the swing trace and correlates the switching times between "positive" and "negative" of the energy change rate with the individual events specified by the event detection part 37.

The image data generation part 33 may be connected to the event calculation part 38 and the timing calculation part 44. The output is supplied from the event calculation part 38 or the timing calculation part 44 to the image data generation part 33. The image data generation part 33 generates image data. In the image data, an image representing the switching times between "positive" and "negative" of the energy change rate with the event information is specified. The image may be a pseudo circular graph in which the switching times between "positive" and "negative" of the energy change rate is specified around the center point, for example.

The arithmetic processing circuit 14 includes a superimposition image data generation part 42. The superimposition image data generation part 42 is connected to the image data generation part 33 and the swing image data generation part 35. In the superimposition image data generation part 42, superimposition image data in which the image data generated in the image data generation part 33 and the image data generated in the swing image data generation part 35 are superimposed is generated. As an example, the energy change rate (analysis information) is superimposed on the swing trace pattern, and thereby, image data for visually representing correlation in time series between the swing trace pattern and the energy change rate is generated. The switching time between "positive" and "negative" of the energy change rate displayed on the swing analysis pattern shows the "positive" and "negative" states of the energy change rate with respect to each event of the swing analysis pattern. For the data representing the state of the energy change rate, swing motion of a professional or an expert may be represented as comparison data. Further, the image data generation part 33 may correlate the evaluation result calculated by the calculation part 32 with the image data for visually representing the state of the energy change rate for display. In this case, in the correlated and displayed image data, graphic display in different colors at times when the positive and negative states of the energy change rate are switched or gradation display with the times when the positive and negative states of the energy change rate are switched may be used.

The arithmetic processing circuit 14 includes a drawing part (display part) 41. The drawing part 41 is connected to the image data generation part 33 and the swing image data generation part 35 via the superimposition image data generation part 42. The output is supplied from the image data generation part 33 and the swing image data generation part 35 via the superimposition image data generation part 42 to the drawing part 41. The drawing part 41 draws an image of the energy change rate (analysis information) according to the time axis via the superimposition image data generation part 42 based on the output of the image data generation part 33. The drawing part 41 draws an image representing the swing motion via the superimposition image data generation part 42 based on the output of the swing image data generation part 35. Further, the drawing part 41 may display the evaluation result of the calculation part 32. By correlation and display of the evaluation result with the image data for visually representing the energy change rate (analysis information), the subj ect may grasp the quality of the swing state at a glance.

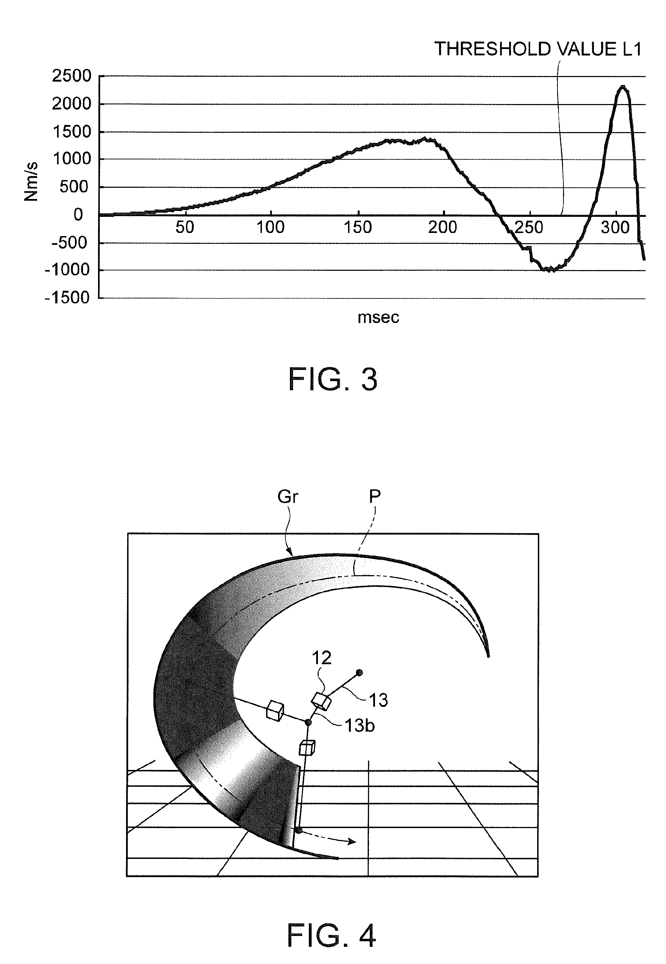

A specific example of the image data displayed by the drawing part 41 will be explained using Figs. 3 and 4. Fig. 3 shows one specific example of a graph showing changes (trend) of the energy change rate according to the time axis with time [ms] on the horizontal axis and the energy change rate on the vertical axis. Note that a threshold value L1 in Fig. 3 is a time axis on which the energy change rate is zero. Fig. 4 shows one specific example of a pseudo circular graph that correlates the changes of the energy change rate and the swing trace.

First, the energy change rate in swing motion is explained with reference to Fig. 3. The energy change rate in swing motion shown in Fig. 3 exemplifies swing as an undesirable example. The horizontal axis in Fig. 3 indicates the moment at which the swing motion comes from the stationary state (top) after backswing (halfway-back) to down-swing as 0 [ms] and the states of subsequent changes of the energy change rate in time series.

As shown in Fig. 3, the energy change rate is in the "positive" state for a while after start of down-swing and tends to increase. Then, before impact, the energy change rate tends to decrease and comes to the "negative" state through the zero-cross point as a point crossing the threshold value L1. Then, the energy change rate tends to increase again toward impact, crosses the threshold value L1 into the "positive" state, and shows the maximum value near impact. The good swing is such that the energy change rate switches to the "negative" state in the relatively early stage before impact, and then, does not return to the "positive" state. This is because the condition means that the total energy change rate in the upper limb of the subject transitions from the positive state to the negative state in the relatively high location during down-swing, and the start of the pendulum motion of the golf club 13 around the joints of the subject at the relatively early stage of the swing and the contribution of the pendulum motion of the golf club 13 to improvement of an energy transfer rate η are predicted. Further, from the condition that the energy change rate once comes from the "positive" state to the "negative" state, and then, returns to the "positive" state again, the attenuation of the pendulum motion of the golf club 13 near impact and reduction of the energy transfer rate η are predicted. Therefore, it is known that the motion is undesirable as swing.

By the observation of the zero-cross of the energy change rate, the time of the specific point in the golf swing that can efficiently transfer energy to a golf ball is derived, and the index of the time of the best swing may be provided. A golfer changes the time of the specific point and repeatedly observes the point of zero-cross or changes (trend) of the energy change rate, and thereby, may improve the golf swing through trial and error. Here, the times of the points in golf swing include not only rhythm and pace of the golf swing but also times of events as points through the golf swing such as the time of relaxation and the time of turn.

As shown in Fig. 4, the changes (trend) of the energy change rate are drawn (displayed) in correlation with the swing trace. In Fig. 4, an image using a pseudo circular graph Gr in a nearly semi-circle shape is drawn for visual representation of the movement trace (swing trace) of the golf club 13 in time series. Then, in the pseudo circular graph Gr drawn on the movement trace (swing trace P) of the golf club 13, the changes (trend) of the energy change rate are drawn by gradation display. In this regard, for example, by display in different colors using red when the change of the energy change rate is "positive" and using blue when the change of the energy change rate is "negative", the state may be visually recognized at a glance. Further, the so-called gradation display of changing shading of colors in response to the magnitudes of the changes (trend) of the energy change rate is effective for improvement of visual recognition. For example, when the change of the energy change rate is larger, the color is darker and, when the change of the energy change rate is smaller, the color is lighter. Note that, as data for comparison, display correlated with the energy change rate and the evaluation result of a professional or an expert may be further superimposed and represented in the drawing.

As described above, the changes (trend) of the energy change rate are superimposed and displayed on the swing trace P and the display is changed with the switching time between "positive" and "negative" of the energy change rate, and thereby, the times when the good or bad states, the positive or negative states, or the like of the trend of the energy change rate are changed may be grasped at a glance. Further, the energy change rate is displayed with the swing trace, and thus, the switching time of the energy provided to the golf club 13 may be grasped at a glance and the state of swing motion and the quality tendency may be precisely determined.

### (4) Operation of Golf Swing Analyzing Apparatus

Returning to Figs. 1 and 2, the operation of the golf swing analyzing apparatus 11 as an example of the motion analyzing apparatus will be briefly explained. First, golf swing of the subject is measured. Prior to the measurement, necessary information is input from the input device 21 to the arithmetic processing circuit 14. Here, according to the three-dimensional motion analysis model 26, input of the location lsj of a supporting point 28 according to the local coordinate system Σs and a rotation matrix R0 of the initial position of the inertia sensor 12 is prompted. The input information is managed under a specific identifier, for example. The identifier may identify a specific subject.

Prior to the measurement, the inertia sensor 12 is attached to the shaft 13a of the golf club 13. The inertia sensor 12 is relatively displaceably fixed to the golf club 13. Here, one of the detection axes of the inertia sensor 12 is aligned with the axis of the shaft 13a. Another one of the detection axes of the inertia sensor 12 is aligned with the hitting direction specified by the direction of the face (hitting surface).

Prior to execution of golf swing, the measurement of the inertia sensor 12 is started. At the start of motion, the inertia sensor 12 is set in predetermined location and position. These location and position correspond to those specified by the rotation matrix R0 of the initial position. The inertia sensor 12 continuously measures the accelerations and the angular velocities at specified sampling intervals. The sampling intervals define the resolution of the measurement. The detection signals of the inertia sensor 12 are sent into the arithmetic processing circuit 14 in real time. The arithmetic processing circuit 14 receives the signals specifying the output of the inertia sensor 12.

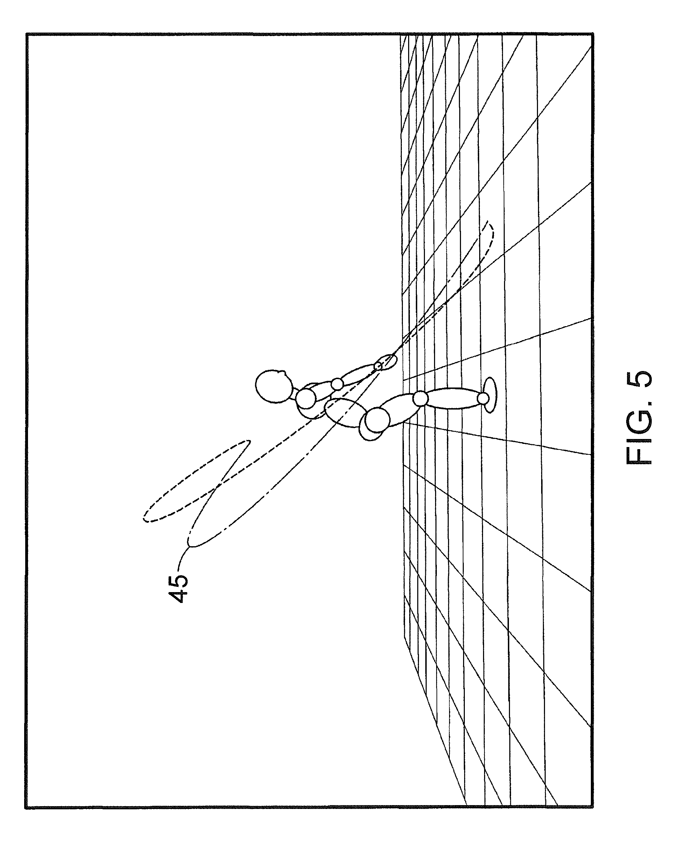

The golf swing starts from address, take-back, halfway-back, top, through down-swing and impact, follow-through, to finish. The position of the shaft 13a at the event such as halfway-back or top corresponds to the second state of the shaft part and corresponds to a dynamic state after the start of swing motion. When the golf club 13 is swung, the position of the golf club 13 changes according to the time axis. The inertia sensor 12 outputs detection signals in response to the position of the golf club 13. Concurrently, the position detection part 34 calculates the positions of the golf club 13 according to the time axis based on the detection signals at swing motion. The swing image data generation part 35 specifies the movement trace (swing trace) of the golf club 13 at swing motion based on the calculated positions of the golf club 13. The swing image data generation part 35 generates three-dimensional image data (e.g., polygon data) visually representing the swing motion. The drawing part 41 draws an image visually specifying the movement trace 45 of the golf club 13 based on the three-dimensional image data as shown in Fig. 5, for example. In this manner, the swing motion is visually represented by the image. The drawing data is sent to the image processing circuit 18 and the image is projected on the screen of the display device 19 according to the drawing data.

For the measurement of golf swing, the subject first assumes the position of address. At the address, the subject reproduces the position at the moment of impact. As a result, the position at the moment of impact is extracted from a series of motion of "golf swing". At the same time, the golf club 13 is held in the stationary position. The stationary state detection part 36 detects the stationary state of the golf club 13. The stationary state detection part 36 outputs the stationary state notification signal. The detection part 31 sets the initial state at the angle "0°" in response to the reception of the stationary state notification signal. The calculation part 32 starts calculation of the energy change rate in response to the reception of the stationary state notification signal. The position detection part 34 starts detection of the position of the golf club 13 in response to the reception of the stationary state notification signal.

Starting from address and during swing motion, the calculation part 32 detects the energy change rates at preset unit time intervals. The energy change rates are specified according to the time axis. The output signals specifying the energy change rates are sent to the image data generation part 33. The image data generation part 33 generates two-dimensional image data specifying an image representing changes of the energy change rates according to the time axis. The drawing part 41 draws the image representing changes of the energy change rates according to the time axis based on the generated two-dimensional image data as shown in Fig. 3, for example. Comparison data of a teaching pro may be drawn on the image at the same time. In addition, the graph of the energy change rates may be projected on the screen on the image of the swing trace shown in Fig. 4. Further, the changes (trend) of the energy change rate and the swing trace may be correlated and drawn (displayed) as shown in Fig. 4. The explanation of the energy change rate is the same as the above described explanation and omitted.

As shown in Fig. 4, the changes (trend) of the energy change rate are drawn (displayed) in correlation with the swing trace P. In the example, the image in which the pseudo circular graph Gr in the nearly semi-circle shape is superimposed is drawn for visual representation of the movement trace (swing trace) P of the golf club 13 in time series. Then, the changes (trend) of the energy change rate are drawn by gradation display in the pseudo circular graph Gr drawn on the movement trace (swing trace) P of the golf club 13. In this regard, for example, by display in different colors using red when the change of the energy change rate is "positive" and using blue when the change of the energy change rate is "negative", the state may be visually recognized at a glance. Further, the so-called gradation display of changing shading of colors in response to the magnitudes of the changes (trend) of the energy change rate is effective for improvement of visual recognition. For example, when the change of the energy change rate is larger, the color is darker and, when the change of the energy change rate is smaller, the color is lighter. Note that, as data for comparison, display correlated with the energy change rate and the evaluation result of a professional or an expert may be further superimposed and represented in the drawing.

According to the golf swing analyzing apparatus, the changes (trend) of the energy change rate are drawn (displayed) on the swing trace P and the display is changed according to the switching time between "positive" and "negative" of the energy change rate, and thereby, the times when the good or bad states, the positive or negative states, or the like of the trend of the energy change rate are changed may be grasped at a glance. Further, the energy change rate is displayed with the swing trace P, and thus, the switching time of the energy provided to the golf club 13 may be grasped at a glance and the state of swing motion and the quality tendency may be precisely determined.

### Second Embodiment

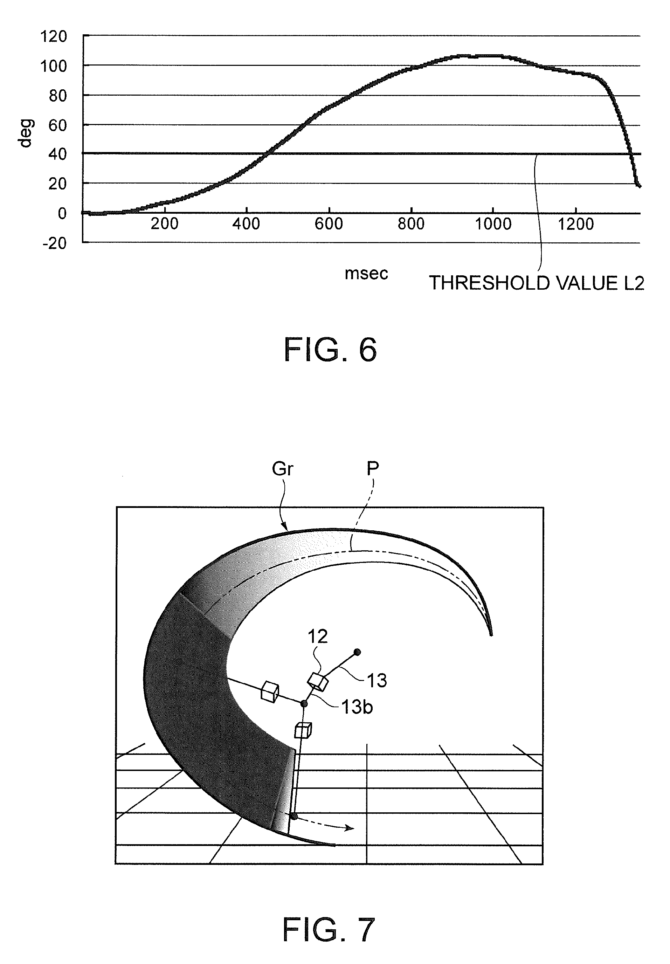

In the above described first embodiment, in the analysis information calculation part 39, the energy change rate indicating the energy state generated between the subject and the golf club 13 from the initial state at the angle "0°" and during swing according to the time axis based on the output of the inertia sensor 12 has been explained as an example. In the second embodiment, in the analysis information calculation part 39, an example of outputting time-series changes of the relative rotation angle θn of the grip 13b during swing from the initial state at the angle "0°" based on the output of the inertia sensor 12 is explained using Figs. 6 and 7. Fig. 6 shows one specific example of a graph showing changes of the relative rotation angle θ according to the time axis in which the time axis [ms] is set on the horizontal axis and the relative rotation angle θn [deg] is set on the vertical axis. Note that, with respect to a threshold value L2 in Fig. 6, the relative rotation angle θn as a determination value for determination of the quality of the relative rotation angle θn is 40 [deg]. Fig. 7 shows one specific example of a pseudo circular graph that correlates the changes of the relative rotation angle θn and the swing trace. The same configurations as those of the above described first embodiment have the same signs and their explanation is omitted.

The motion analyzing method according to the second embodiment includes: calculating location information containing the swing trace in swing motion in time series using the output of the inertia sensor 12; calculating the relative rotation angle θn of the grip 13b in the swing motion in time series as analysis information using the output of the inertia sensor 12 ; acquiring the switching time with respect to a set threshold value; and displaying the swing trace with the state of the relative rotation angle θn of the grip 13b. Further, the above described displaying includes changing the display of the swing trace with the switching time. Specifically, the changes of the relative rotation angle θn of the grip 13b obtained as the analysis information are drawn on the swing trace using gradation display or the like according to the switching times.

The calculation part 32 includes the analysis information calculation part 39 and the swing trace calculation part 40. The analysis information calculation part 39 detects the relative rotation angles θn (n = 1, ..., N) of the grip 13b around the longitudinal axis of the shaft 13a during swing from the initial state at the angle "0°" based on the output of the inertia sensor 12. For the detection, the calculation part 32 calculates an amount of change of the rotation angle per unit time and the calculated amounts of change are accumulated. Here, N is the number of samples (the same applies to the following description) . Then, the amount of change from the initial state is calculated with respect to each time of accumulation per unit time. In this manner, the relative rotation angle θn of the grip 13b is specified according to the time axis. The swing trace calculation part 40 calculates the trace of the shaft 13a during swing (swing trace) from the initial state at the angle "0°" based on the output of the inertia sensor 12. For the detection, the swing trace calculation part 40 calculates the trace of the swing according to the time axis (swing trace in time series) in the three-dimensional space using the above described motion analysis model based on the output of the inertia sensor 12. Note that the swing trace includes the trace of the shaft 13a, the movement trace of the subject itself, or the like.

Further, in the calculation part 32, an evaluation of quality of the swing state is performed using the specified relative rotation angle θn. In the evaluation, comparison with the preset threshold value L2 of the relative rotation angle θn is made. For example, if the relative rotation angle θn exceeds the threshold value L2, generation of undesirable twist is evaluated and, if the relative rotation angle θn does not exceed the threshold value L2, smooth swing without generation of undesirable twist is evaluated. Therefore, the threshold value L2 in the second embodiment is a quality determination value of the relative rotation angle θn of the grip 13b. Note that, in the example of the graph in Fig. 6, the example in which the threshold value L2 is set to 40° is shown. In the example shown in the drawing, the relative rotation angle θn of the grip 13b in the swing of the subject is largely above 40°. This shows that the amount of rotation around the shaft axis is larger, i.e., the amount of twist is larger in the swing. When the amount of twist is larger, it is necessary to reverse the twist so that the face of the club head may be horizontal with respect to the target line (hitting direction) at impact, and the swing is not good. The subject may grasp at which point of the swing motion the amount of twist is larger according to the presented evaluation result, and improve the form of the swing. Note that one threshold value L2 is displayed in Fig. 6, however, a plurality of threshold values may be set. For example, threshold values at a plurality of stages are set according to the technique of the subject so that the subject may feel senses of achievement in stages.

Further, as shown in Fig. 7, the changes of the relative rotation angle θn of the grip 13b are drawn (displayed) in correlation with the swing trace P. In Fig. 7, an image with a pseudo circular graph Gr in a nearly semi-circle shape is drawn for visual representation of the movement trace (swing trace) P of the golf club 13 in time series. Then, in the pseudo circular graph Gr drawn on the movement trace (swing trace) P of the golf club 13, the changes (trend) of the relative rotation angle θn of the grip 13b are drawn by gradation display. In this regard, for example, by display in different colors using red when the relative rotation angle θn of the grip 13b is larger than the threshold value L2 and using blue when the relative rotation angle θn of the grip 13b is smaller than the threshold value L2, the state may be visually recognized at a glance. Further, the display of the magnitudes of the changes (trend) of the energy relative rotation angle θn of the grip 13b in shading of colors is effective for improvement of visual recognition. For example, when the change of the relative rotation angle θn of the grip 13b is larger, the color is darker and, when the change of the relative rotation angle θn of the grip 13b is smaller, the color is lighter. Note that, as data for comparison, display correlated with the relative rotation angle θn and the evaluation result of a professional or an expert may be further superimposed and represented in the drawing.

As described above, the changes (trend) of the relative rotation angle θn of the grip 13b are superimposed and displayed on the swing trace and the display is changed with the switching time between good and bad with respect to the threshold value L2 of the relative rotation angle θn of the grip 13b, and thereby, the change tendency of the relative rotation angle θn of the grip 13b or the time when the change of the relative rotation angle θn of the grip 13b is switched between good and bad may be grasped at a glance during swing motion. Further, the state of change of the relative rotation angle θn of the grip 13b is displayed with the swing trace P, and thus, the state of the relative rotation angle θn of the grip 13b may be grasped at a glance in correspondence with the time-series swing trace and the state of swing motion and the quality tendency may be precisely determined.

### Third Embodiment

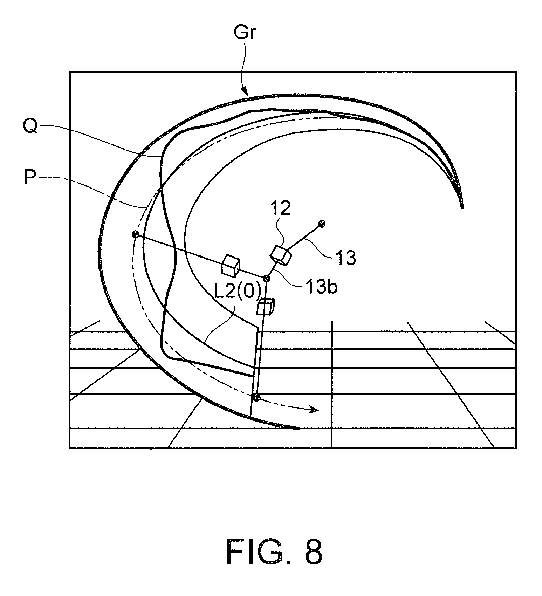

In the above described first embodiment, the example in which the energy change rates (trend) in swing motion are drawn by gradation display in the pseudo circular graph in the nearly semi-circle shape drawn on the movement trace (swing trace) of the golf club 13 has been explained, however, the drawing is not limited to that. In the third embodiment, the energy change rates (trend) are drawn by a line graph in the pseudo circular graph Gr in the nearly semi-circle shape drawn on the movement trace (swing trace) of the golf club 13 in place of the gradation display. As below, the third embodiment will be explained using Fig. 8. Fig. 8 shows one specific example of a pseudo circular graph that correlates the changes of the energy change rate with the swing trace according to the third embodiment. The same configurations as those of the above described first embodiment have the same signs and their explanation is omitted.

The motion analyzing method according to the third embodiment includes: calculating location information containing the swing trace in swing motion in time series using the output of the inertia sensor 12; calculating the energy change rate in the swing motion in time series as analysis information using the output of the inertia sensor 12; acquiring the switching time with respect to a set threshold value; and displaying the swing trace with the energy change rate. Further, the above described displaying includes changing the display of the swing trace with the switching time . Specifically, the changes of the energy change rate obtained as the analysis information are drawn with the switching times by a line graph using lines in the pseudo circular graph in the nearly semi-circular shape drawn on the movement trace of the golf club 13 (swing trace).

Specifically, like the above described first embodiment, in the golf swing analyzing method (motion analyzing method) according to the third embodiment, in the timing acquisition part 43 contained in the arithmetic processing circuit 14, of the energy change rates calculated in time series, the times when the energy change rate is switched to "positive" or "negative" are acquired. The threshold value in this regard is set on an axis on which the positive state and the negative state of the energy change rate are switched, i.e., the time axis on which the energy change rate is zero as described above.

The arithmetic processing circuit 14 includes a timing calculation part 44. The timing calculation part 44 is connected to the timing acquisition part 43 and the event calculation part 38. The output of the timing acquisition part 43 and the output of the event calculation part 38 are supplied to the timing calculation part 44. The timing calculation part 44 correlates the switching times between "positive" and "negative" of the analysis information (energy change rate) with the swing trace and correlates the switching times between "positive" and "negative" of the analysis information (energy change rate) with the individual events specified by the event detection part 37.

Like above described first embodiment, the output is supplied from the event calculation part 38 or the timing calculation part 44 to the image data generation part 33, and the part generates image data. In the image data, an image representing the switching times between "positive" and "negative" of the analysis information (energy change rate) with the event information is specified. The image may be a pseudo circular graph in which the switching times between "positive" and "negative" of the energy change rate are specified around the center point, for example.

The arithmetic processing circuit 14 includes a superimposition image data generation part 42. The superimposition image data generation part 42 is connected to the image data generation part 33 and the swing image data generation part 35. In the superimposition image data generation part 42, superimposition image data in which the image data generated in the image data generation part 33 and the image data generated in the swing image data generation part 35 are superimposed is generated. As an example, the energy change rate (analysis information) is superimposed on the swing trace pattern, and thereby, image data for visually representing correlation between the swing trace pattern and the energy change rate is generated. The switching time between "positive" and "negative" of the energy change rate displayed on the swing analysis pattern shows the "positive" and "negative" states of the energy change rate with respect to each event of the swing analysis pattern. For the image data for visually representing the states of the energy change rate, a line graph using lines from which the times when the positive and the negative states of the energy change rate are switched are known is displayed in the graphical representation. Furthermore, swing motion of a professional or an expert may be represented as comparison data. Further, the image data generation part 33 may correlate the evaluation result calculated by the calculation part 32 with the image data for visually representing the state of the energy change rate for display.

The arithmetic processing circuit 14 includes a drawing part (display part) 41. A specific example of image data displayed by the drawing part 41 will be explained with reference to Fig. 8. Note that the energy change rate in swing motion is the same as that of the above described first embodiment, and the detailed explanation will be omitted.

As shown in Fig. 8, the changes (trend) of the energy change rate are drawn (displayed) in correlation with the swing trace. In the drawing shown in Fig. 8, an image in which a pseudo circular graph Gr in a nearly semi-circle shape is superposed is drawn for visual representation of the movement trace (swing trace) P of the golf club 13 in time series. Then, in the pseudo circular graph Gr drawn on the movement trace (swing trace) P of the golf club 13, the changes (trend) of the energy change rate are drawn by lines Q. In this regard, for example, by display in different thickness of lines using a thick line when the change of the energy change rate is "positive" and using a thin line when the change of the energy change rate is "negative", the state may be easily visually recognized at a glance. Note that, as data for comparison, display correlated with the energy change rate and the evaluation result of a professional or an expert may be further superimposed and represented in the drawing.

As described above, the changes (trend) of the energy change rate are superimposed and displayed on the swing trace P and the display is changed with the switching time between "positive" and "negative" of the energy change rate, and thereby, the times when the good or bad states, the positive or negative states, or the like of the trend of the energy change rate are changed may be grasped at a glance. Further, the energy change rate is displayed with the swing trace P, and thus, the switching time of the energy provided to the golf club 13 may be grasped at a glance and the state of swing motion and the quality tendency may be precisely determined.

Note that, in the third embodiment, the example of displaying the energy change rate has been explained, however, the line graph using lines showing the changes of the relative rotation angle θn of the grip 13b explained in the second embodiment may be employed.

As above, the embodiments have been explained in detail, however, a person skilled in the art could readily understand that many modifications may be made without substantially departing from the new matter and the advantages of the invention. Therefore, these modified examples may fall within the range of the invention. For example, in the specification and the drawings, terms described with different terms in a broader sense or synonymous sense at least once may be replaced by the different terms in any part of the specification or drawings. Further, the configurations and operations of the inertia sensor 12, the arithmetic processing circuit 14, the three-dimensional motion analysis model 26, etc. are not limited to those explained in the embodiments, but various modifications may be made. Furthermore, the invention can be applied not only to the golf swing but also to the sports using sporting tools such as tennis and baseball.

## Claims

1. A motion analyzing method comprising:
acquiring location information of at least one of a sporting tool and a subject in swing motion in time series based on an inertial force generated by the swing motion;
acquiring analysis information of the swing motion based on the inertial force;
acquiring a time when the analysis information is switched with respect to a preset threshold value; and
correlating information of the time with the location information and outputting the information.

2. The motion analyzing method according to claim 1,
wherein the analysis information is position data of the sporting tool in the swing motion.

3. The motion analyzing method according to claim 1 or claim 2, wherein the outputting includes correlating the information of the time with a trace of the swing motion drawn in time series based on the location information and outputting the information.

4. The motion analyzing method according to claim 3,
wherein display of the trace is changed between before and after the time.

5. The motion analyzing method according to any of claims 1 to 4, further comprising determining a state change of swing before and after the time,
wherein the outputting includes changing display of the trace in response to the state change.

6. The motion analyzing method according to any of claims 1 to 5, further comprising determining quality of swing before and after the time,
wherein the outputting includes changing display of the trace in response to the quality.

7. A motion analyzing method comprising:
acquiring location information of at least one of a sporting tool and a subject in swing motion in time series based on an inertial force generated by the swing motion;
acquiring analysis information of the swing motion based on the inertial force and generating a graph of the analysis information; and
correlating information of the graph with a trace of the swing motion drawn in time series based on the location information and outputting the information.

8. A motion analyzing apparatus comprising:
a unit that acquires location information of at least one of a sporting tool and a subject in swing motion in time series based on an inertial force generated by the swing motion;
a unit that acquires analysis information of the swing motion based on the inertial force;
a unit that acquires a time when the analysis information is switched with respect to a preset threshold value; and
a unit that correlates information of the time with the location information and outputs the information.

9. The motion analyzing apparatus according to claim 8, further comprising a unit that correlates the information of the time with a trace of the swing motion drawn in time series based on the location information and outputs the information.

10. The motion analyzing apparatus according to claim 8 or claim 9, wherein display of the trace is changed between before and after the time.
